Europäisches Patentamt

**European Patent Office**

Office européen des brevets

(19)

(11) Publication number: **0 255 394**
**A2**

## (12) EUROPEAN PATENT APPLICATION

(21) Application number: **87306787.0**

(22) Date of filing: **31.07.87**

(51) Int. Cl.⁴: **C 07 K 7/10**
**A 61 K 37/02**

(30) Priority: **01.08.86 US 892588**

(43) Date of publication of application:
**03.02.88 Bulletin 88/05**

(84) Designated Contracting States:
**AT BE CH DE ES FR GB GR IT LI LU NL SE**

(71) Applicant: **Schering Biotech Corporation**
**901 California Avenue**
**Palo Alto California 94304-1104 (US)**

(72) Inventor: **Martens, Christine Lee**
**141 Erica Way**
**Menlo Park California 94025 (US)**

**Moore, Kevin William**
**4144 Park Boulevard**
**Palo Alto California 94306 (US)**

(74) Representative: **Ritter, Stephen David et al**
**Mathys & Squire 10 Fleet Street**
**London EC4Y 1AY (GB)**

(54) **Immunosuppressive peptides.**

(57) Immunosuppressive peptides having glycosylation inhibiting factor activity are provided. The peptides include the following sequence and its homologs:

H - Cys - Val - Cys - Val - Cys - Leu - Leu - Pro - Arg - Tyr - Pro - - Ser - Ala - Gly - Val - Phe - Thr - Tyr - Leu - Asn - Thr - Lys - Ile - Ile - Thr - Phe - Asp - Ser - Val - Leu - Ser - Lys - Cys - Ala - - OH.

EP 0 255 394 A2

**Description**

## IMMUNOSUPPRESSIVE PEPTIDES

The present invention relates generally to compounds capable of suppressing immune responses, and more particularly to so-called glycosylation inhibiting factors, which are capable of causing the suppression of immunoglobulin E (IgE) production.

As far as can be determined, the main physiological function of IgE-mediated responses is to combat parasites. The response can be divided into five phases: an IgE-bearing B cell is stimulated to respond to an antigen (phase I) and activated to secrete IgE antibodies (phase 2); the antibodies produced bind to mast cells and basophils in tissues (phase 3, antibody fixation); then interaction of allergen with cell-bound IgE activates these cells and causes the release of chemical mediators stored in their granules (phase 4, degranulation); and finally, the mediators induce a complex tissue response aimed at the elimination of nonmicrobial parasites from the body (phase 5). Part of this defense mechanism is an attack on the tissue that harbors the parasite--that is, on self. To excise a parasite from a tissue without damaging the rest of the body is an extraordinarily delicate act. The mediators released by activated mast cells and basophils can cause considerable harm, even death, if released at an inappropriate time or if directed at an inappropriate target. The IgE response must be closely controlled and quickly attenuated after its goal has been achieved. As long as this control is functioning there is no danger that healthy parts of the body will be damaged, but, should the controls fail, the beneficial reaction will turn into a harmful one. About 90 percent of all humans have no difficulty in using their IgE only for defensive purposes; but the remaining unlucky l0 percent carry a genetic defect of the control mechanism that permits the stimulation of IgE-responses by antigens that have nothing to do with parasites. At first it was thought that this defect was limited only to humans, but similar defects were discovered later in several other mammals. The inappropriately stimulated IgE-responses cause a plethora of diverse diseases, grouped under the name allergy or atopy: Klein, Immunology: The Science of Self-Nonself Discrimination (John Wiley & Sons, New York, 1982).

Currently glucocorticoid steroids are the most effective drugs for treating allergic diseases. However, prolonged steroid treatment is associated with many deleterious side effects: Goodman and Gillman, The Pharmacological Basis of Therapeutics, 6th ed. (MacMillan Publishing Company, New York, 1980). Recently, Ishizaka and his co-workers have discovered and characterized a class of compounds which they have designated as glycosylation-inhibiting factors (GIFs): Ishizaka, Ann. Rev. Immunol., Vol. 2, pgs. 159-182 (1984). GIFs are natural compounds which are capable of causing T cells to produce a class of IgE-binding factors which, in turn, selectively suppress the IgE response (IgE-suppressive factors). Apparently, one GIF is related to lipomodulin, a phospholipase-inhibitory protein, which is induced by treatment of a variety of cells with glucocorticoid, e.g. Flower et al., Nature, Vol. 278 pgs 456-459 (1979). This GIF inhibits the assembly of N-linked oligosaccharide to IgE-binding factors for the selective formation of IgE-suppressive factors. It suppresses IgE-induced expression of Fc-epsilon receptors on lymphocytes; the factor has a molecular weight of l5-l6 kilodaltons, and it binds to monoclonal antibodies against lipomodulin: Uede et al., J. Immunol., Vol. l30, pgs 878-884 (1983), and Iwata et al., J. Immunol., Vol. l32, pgs. 1286-1294 (1984). Wallner et al., in Nature Vol. 320, pgs. 77-8I (1986) report the cloning, sequencing and expression of human lipocortin (lipocortin is another name for lipomodulin).

In view of the foregoing it would be advantageous to develop further immunosuppressive compounds, particularly GIFs, which are effective in reducing or eliminating inappropriate IgE responses, but which do not significantly introduce unwanted side effects.

The present invention provides a group of immunosuppressive peptides whose amino acid sequences are derived from a native nucleotide sequence capable of expressing GIF activity. The immunosuppressive peptides of the invention are defined by the formula (of the reduced form):

H - Cys - X(Val) - Cys - X(Val) - Cys - X(Leu) - X(Leu) - X(Pro) - X(Arg) - X(Tyr) - X(Pro)
Ser - X(Ala) - Gly - X(Val) - X(Phe) - Thr - X(Tyr) - X(Leu) - X(Asn) - Thr - X(Lys) - X(Ile) - X(Ile) - Thr - X(Phe) --
X(Asp) - Ser - X(Val) - X(Leu) - Ser - X(Lys) - Cys - X(Ala) - OH

Formula I

wherein the term X(Xaa) represents the group of synonymous amino acids to the amino acid Xaa;
together with the 2-fold inserted and/or 2-fold deleted peptides of formula I;
and the partially and fully oxidized forms thereof.

Synonymous amino acids within a group have sufficiently similar physiochemical properties for substitution between members of the group to preserve substantially the biological function of the peptide: Grantham, Science, Vol. l85, pgs 862-864 (1974). It is clear that insertions and deletions of amino acids may also be made in the above-defined sequence without altering biological functions, particularly if the insertions of deletions are of only a few amino acids, e.g. one or two, and do not remove or displace amino acids which are critical to a functional conformation: Anfinsen, Science, Vol. l8l, pgs 223-230 (1973). Peptides differing from those of Formula I by such minor deletions or insertions come within the purview of the present invention.

Amino acid nomenclature recommended by the following references is used throughout: Cohn, Methods in Enzymology, Vol. l06, pgs. 3-I7 (Academic Press, New York, 1984); and IUPAC-IUB Commission, J. Bio. Chem.,

Vol. 247, pgs. 977-983 (1972).

The partially oxidized forms of the polypeptides of formula I have one S-S bridge between cysteine residues therein, and the fully oxidized forms have two such S-S bridges.

Preferably the synonymous amino acid groups are those defined by Table I, and more preferably they are those defined in Table II:

## Table I. Preferred Groups of
## Synonymous Amino Acids

| Amino Acid | Synonymous Group |
|---|---|
| Arg | Arg, His, Lys |
| Leu | Leu, Ile, Phe, Met |
| Pro | Pro, Ala |
| Ala | Ala, Pro |
| Val | Val, Met, Ile |
| Ile | Ile, Met, Phe, Val, Leu |
| Phe | Phe, Met, Tyr, Ile, Leu |
| Tyr | Tyr, Phe |
| Asn | Asn, Asp |
| Lys | Lys, Arg |
| Asp | Asp, Asn |

## Table II. More Preferred Groups of
## Synonymous Amino Acids

| Amino Acid | Synonymous Group |
|---|---|
| Arg | Arg |
| Leu | Leu, Ile, Met |
| Pro | Pro |
| Ala | Ala |
| Val | Val |
| Ile | Ile, Met, Leu |
| Phe | Phe |
| Tyr | Tyr |
| Asn | Asn |
| Lys | Lys |
| Asp | Asp |

Most preferably the peptide of the invention is defined by the following sequence of amino acids:

H - Cys - Val - Cys - Val - Cys - Leu - Leu - Pro - Arg - Tyr - Pro - Ser - Ala - Gly - Val - Phe - Thr - Tyr - Leu - - Asn - Thr - Lys - Ile - Ile - Thr - Phe - Asp - Ser - Val - Leu - Ser - Lys - Cys - Ala - OH

Formula II

The invention includes peptides of Formula I with amino acid substitutions (of a synonymous amino acid for an amino acid in Formula II) at a single position or at multiple positions. The term "N-fold substituted" in reference to the peptides of Formula I is used to describe a subset of peptides wherein synonymous amino acids have been substituted for the amino acids of Formula II at at most N positions. Thus, for example, the group of I-fold substituted peptides of Formula I consists of 48 peptides for the preferred groups of synonymous amino acids, and I2 peptides for the more preferred groups of amino acids.

Likewise, the term "N-fold inserted" in reference to the peptides of Formula I is used to describe a set of peptides wherein up to N amino acids have been inserted into the sequence defined by Formula I, where N is 2. Preferably, the inserted amino acids are selected from the preferred groups of synonymous amino acids (Table I) of either amino acid flanking the insertion; more preferably they are selected from the more preferred groups of synonymous amino acids (Table II) of either amino acid flanking the insertion. Thus, for example, one subgroup of the group of 2-fold inserted peptides comprises two amino acids inserted between the N-terminal X(Pro) and the adjacent X(Arg). The insertions defining the members of this subgroup are preferably selected from Pro, Ala, Arg, His, and Lys; and more preferably they are selected from Pro and Arg. Insertions can be made between any adjacent amino acids of Formula I. Since there are 33 possible insertion locations, and since multiple insertions can be made at the same location, a 2-fold inserted peptide of Formula I gives rise to I089 subgroups of peptides, and the size of each subgroup depends on the sizes of the synonymous amino acid groups of the amino acids flanking the insertions.

The term "N-fold deleted" in reference to the peptides of Formula I is used to describe a set of peptides having up to N amino acids deleted from the sequence defined by Formula I, where N is 2. Thus, the set of I-fold deleted peptides of Formula I consists of 34 subgroups of peptides each 33 amino acids in length (33-mers). Each of the subgroups in turn consists of all the 33-mers defined by the preferred, or more preferred, synonymous amino acid groups.

The invention also includes pharmaceutical compositions comprising one or more peptides of the invention, or their pharmaceutically acceptable salts, and suitable carrier compounds.

The present invention is directed to problems associated with abnormal and/or allergic immune responses involving the production of IgE. In particular, new low molecular weight GIFs are provided which cause T cells of the immune system to preferentially secrete IgE-suppressive factors which, in turn, reduce the intensity of an IgE-mediated immune response.

The invention also provides a process for the preparation of a peptide of formula I, or of a 2-fold inserted and/or 2-fold deleted peptide thereof, or of a partially or fully oxidised form thereof, which comprises the steps of:

1) providing a base-support with a functional group to which the first amino acid is anchored;
2) coupling the next amino acid, protected, to build up the polypeptide chain;
3) deprotecting as necessary said next amino acid;
4) repeating steps 2) and 3) until the full polypeptide chain is assembled; and
5) cleaving the complete polypeptide chain from the base support.

Whereas either the carboxy group or the amino group of the amino acid can be anchored to the base support, it is normally more convenient to attach the carboxy group. This will usually have been effected in two preliminary steps: a step of anchoring the first amino acid, protected, to said functional group of the base-support; and then deprotecting said first amino acid. Each deprotecting step can include two associated substeps: a sub-step of neutralization before the next coupling step; and a sub-step of "capping" any terminal amino acid (or the functional group of the base-support) from the previous deprotecting step that failed to react in the intermediary coupling step. Any such "capped" amino acid chain is already one amino acid unit too short, and is thus prevented from reacting further and producing undesired by-products. The coupling step and all other steps can be carried out under standard conditions. The step of cleaving can include three associated sub-steps: a sub-step of deprotecting, a sub-step of partially or completely oxidizing to introduce one or two S-S bonds; and a sub-step of purifying the final polypeptide. The sub-step of deprotecting and oxidizing may be carried out before or after the step of cleaving; indeed, the sub-step of deprotecting can often be carried out simultaneously with the step of cleaving when a single reagent will effect both reactions. The sub-step of oxidizing often occurs spontaneously.

Peptides of the invention are synthesized by standard techniques, e.g. Stewart and Young, Solid Phase Peptide Synthesis, 2nd Ed. (Pierce Chemical Company, Rockford, IL, I984). Preferably a commercial automated synthesizer is used, e.g. Vega Biochemicals (Tucson, AZ) models 296A or B, or Applied Biosystems, Inc. (Foster City, CA) model 430A.

The protected peptide of Formula II was conveniently assembled by solid phase synthesis on a cross-linked polystyrene support starting from the carboxyl terminal residue and adding amino acids in a stepwise fashion until the entire 34-residue chain had been formed. The synthesis was performed on a fully automated peptide synthesizer (Applied Biosystems, Inc. model 430A). The following references are guides to the chemistry employed during synthesis: Merrifield, J. Amer. Chem. Soc., Vol. 85, pg. 2I49 (I963); Kent et al., pg I85, in Peptides I984, Ragnarsson, Ed. (Almquist and Weksell, Stockholm, I984); Kent et al., pg. 2I7 in Peptide

Chemistry 84, Izumiya, Ed. (Protein Research Foundation, B.H. Osaka, 1985); Merrifield, Science, Vol. 232, pgs. 341-347 (1986); and references cited in this last reference.

In solid state synthesis it is most important to eliminate synthetic by-products, which are primarily termination, deletion, or modification peptides. Most side reactions can be eliminated or minimized by use of clean, well-characterized resins, clean amino acid derivatives, clean solvents, and the selection of proper coupling and cleavage methods and reaction conditions: e.g. Barany and Merrifield, The Peptides, Cross and Meienhofer, Eds., Vol. 2, pgs 1-284 (Academic Press, New York, 1979). It is important to monitor coupling reactions to determine that they proceed to completion so that deletion peptides missing one or more residues will be avoided. The quantitative ninhydrin reaction is useful for that purpose: Sarin et al., Anal. Biochem, Vol. 117, pg 147 (1981). Nα-t-butyloxycarbonyl-amino acids (t-Boc-amino acids) were used with appropriate side-chain protecting groups stable to the conditions of chain assembly but labile to strong acids. After assembly of the protected peptide chain, the protecting groups were removed and the peptide anchoring bond was cleaved by the use of low and then high concentrations of anhydrous hydrogen fluoride in the presence of a thioester scavenger: Tam et al., J. Amer. Chem. Soc., Vol. 105, pg. 6442 (1983).

Side-chain protecting groups used (given in parentheses after the amino acid) were Asp(OBzl), Glu(OBzl), Ser(Bzl), Thr(Bzl), Lys(Cl-Z), Tyr(Br-Z), Arg(NGTos), Cys(4-MeBzl), and His(ImDNP). (Protecting groups: Bzl, benzyl; Tos, toluenesulfonyl; DNP, dinitrophenyl; Im, imidazole; Z, benzyloxycarbonyl). The remaining amino acids had no side-chain protecting groups. All the amino acids were obtained from Peninsula Laboratories, except the t-Boc-His(ImDNP), which was from Chemical Dynamics and was recrystallized from ethanol before use. For each cycle the t-Boc-Nα-protected peptide-resin was exposed to 65 percent trifluoroacetic acid (from Eastman Kodak) (distilled before use) in dichloromethane (DCM) (Mallinckrodt): first for 1 minute and then for 13 minutes to remove the Nα-protecting group. The peptide-resin was washed in DCM and then neutralized twice with 10 percent diisopropylethylamine (DIEA) (Aldrich) in dimethylformamide (DMF) (Applied Biosystems), for 1 minute each. Neutralization was followed by washing with DMF. Coupling was performed with the preformed symmetric anhydride of the amino acid in DMF for 16 minutes. The preformed symmetric anhydride was prepared on the synthesizer by dissolving 2 mmol of amino acid in 6 ml of DCM and adding 1 mmol of dicyclohexycarbodiimide (Aldrich) in 2 ml of DCM. After 5 minutes, the activated amino acid was transferred to a separate vessel and the DCM was evaporated off by purging with a continuous stream of nitrogen gas. The DCM was replaced by DMF (6 ml total) at various stages during the purging. After the first coupling, the peptide-resin was washed with DCM, 10 percent DIEA in DCM, and then with DCM. For recoupling, the same amino acid and the activating agent, dicyclohexylcarbodiimide, were transferred sequentially to the reaction vessel. After activation in situ and coupling for 10 minutes, sufficient DMF was added to make a 50 percent DMF-DCM mixture, and the coupling was continued for 15 minutes. Arginine was coupled as a preformed ester of hydroxybenzotriazole (Aldrich) in DMF for 60 minutes and then recoupled in the same manner as the other amino acids. Asparagine and glutamine were coupled twice as preformed hydroxybenzotriazole esters in DMF, 40 minutes for each coupling. For all residues, the resin was washed after the second coupling and a sample was automatically taken for monitoring residual uncoupled α- amine by quantitative ninhydrin reaction (Sarin et al., cited above).

Standard assays for GIF activity are used to test the synthetic peptides: e.g. Iwata et al., J. Immunol., Vol. 132, pgs. 1286-1293 (1984); Uede et al., J. Immunol., Vol. 130, pgs. 878-884 (1983); Akasaki et al., J. Immunol., Vol. 136, pgs. 3172-3179 (1986), and Jardieu et al., J. Immunol., Vol. 133, pgs. 3266-3273 (1984).

GIF is assayed (1) by its ability to switch T cells from producing IgE-binding factors having potentiating activity (IgE-PF) to producing IgE-binding factors having suppressive activity (IgE-SF), or (2) by its ability to inhibit IgE-induced expression of Fc-epsilon receptors on lymphocytes. Below, assays applicable to rodent GIFs are described. Some routine experimentation may be required to develop analogous assays for other mammalian species. It is believed that the biological effects of GIFs are interspecific. For example, rat GIF from 23B6 cells (deposited with the American Type Culture Collection, Rockville, MD, under accession number HB8521) have been shown to switch human T cell hybridoma 166A2 (described by Huff and Ishizaka in Proc. Natl. Acad. Sci., Vol. 81, pg. 1514 [1984]) from the production of IgE-PF to the production of IgE-SF.

One method for assaying the ability of GIF to switch T cells from IgE-PF to IgE-SF production depends on the observation that the differences between IgE-PF and IgE-SF reside in their respective states of glycosylation, and that these differences can be detected by affinity for certain lectins: Martens et al., Proc. Natl. Acad. Sci., Vol. 82, pgs. 2460-2464 (1985). IgE-PF has affinity for lentil lectin and concanavalin A, whereas IgE-SF fails to bind to either lectin: Yodoi et al., J. Immunol., Vol. 128, pg. 289 (1982). Thus, for example, normal BALB/c spleen cells can be used to assay GIF activity as follows. Samples of normal spleen cells (about $1 \times 10^7$ nucleated cells/ml) are cultured for about 24 hours with 10 micrograms/ml mouse IgE in the presence or absence (controls) of the samples to be tested, and the IgE-binding factors formed in the cultures are fractionated on a lentil-lectin Sepharose column. When normal BALB/c spleen cells are cultured with IgE alone, IgE-binding factors formed by the cells are distributed approximately equally between the effluent and eluate fractions. The majority of IgE-binding factors formed in the presence of a GIF should fail to bind to the lentil-lectin Sepharose.

IgE-binding factors in either the effluent or eluate fractions are measured by their ability to inhibit rosette formation between cells having Fc-epsilon receptors and IgE-coated erythrocytes. Red cells coated with human serum albumin (HSA) are used to indicate the level of nonspecific rosettes. IgE-coated erythrocytes are prepared by the method disclosed by Gonzalez-Molina et al., J. Clin. Invest., vol. 59, pg. 616 (1977), with

6

slight modification as disclosed by Yodoi and Ishizaka, J. Immunol., Vol. 122 pg. 2579. Briefly, ox erythrocytes (Colorado Serum Co., Denver, Colo.) are treated successively with trypsin (Miles Laboratories, Elkhart, Ind.), pyruvic aldehyde (ICN Pharmaceutical Inc., Plainview, N.Y.), and formaldehyde, and the fixed cells are kept in phosphate-buffered saline (PBS), pH 7.2. Sensitization of fixed erythrocytes with human IgE, rat IgE, or human serum albumin (HSA, 2 × recrystallized, Nutritional Biochemicals, Cleveland, Ohio) is carried out in 0.1 M acetate buffer, pH 5.0. A 4% suspension of fixed erythrocytes is mixed with an equal volume of a protein solution of an appropriate concentration, and the suspensions are rotated for 2 hr at room temperature. An optimal cncentration of each protein for sensitization is determined by preliminary experiments. Preferably, about 0.2 mg/ml of human IgE, 0.25 mg/ml of rat IgE, and 0.25 mg/ml of HSA is employed to sensitize the cells. A 1% suspension of the sensitized cells in PBS is stored at 4°C, and the cells are used for rosette formation within 1 week after coupling.

Mesenteric lymph node (MLN) cells from a rat (e.g. Lewis strain, available from Microbiological Associates) infected with the nematode Nippostrongylus brasiliensis (Nb) can be the source of Fc-epsilon positive cells for the assay. For example, a rat can be infected with 2800-3000 larvae of Nb via a subcutaneous route as described by Ogilvie: Nature, Vol. 204, pg. 91 (1964). MLN cells are obtained 2-3 weeks after infection using standard procedures, e.g. Ishizaka et al., Cell. Immunol., Vol. 22, pg. 248 (1976).

The rosette inhibition assay proceeds as follows. About 20 microliters of suspended MLN cells (about 5 × $10^6$/ml) are incubated for about 10 minutes at 37°C with an equal volume of a 1% suspension of fixed erythrocytes coated with IgE (or HSA). The cell suspension is then centrifuged at about 90 × G for 5 minutes, and kept at 0°C for 90 minutes. Pellets are gently mixed with 0.1% toluidine blue in PBS and then examined in a hemocytometer. At least 300, and preferably 600-1000, cells are counted for enumerating the percentage of rosette-forming cells (RFCs). A positive RFC is defined as a cell having at least three fixed erythrocytes adhering to its surface. HSA-coated fixed erythrocytes are used to quantify the degree of nonspecific rosette formation.

GIFs can also be assayed by direct tests of the IgE-SF activity of the binding factors produced in its presence. A source of binding factors is T cells primed with an antigen known to preferentially elicit an IgE response, e.g. dinitrophenylated ovalbumin (DNP-OA). (Thus, the IgE-BFs from these sources are presumed to have IgE-PF activity, which in the presence of GIF will be switched to IgE-SF activity.) As mentioned below, an alternative source of IgE-PFs is supernatants from Cos7 cells transfected with the plasmid 23B6p8.3, described by Martens et al. (cited above) and deposited with the ATCC under accession number 39633. The suppressive effects of the binding factors are tested on cells which have been induced to produce IgE. In a rodent system, cultures of DNP-OA primed rat MLN cells serve as a source of IgE-producing cells. The MLN cells are suspended in Click's medium (Cell Immunol., Vol. 3, pg. 264 [1972]) supplemented with 10 percent normal rat serum, 5 × $10^{-5}$M mercaptoethanol, 100 U/ml of penicillin, and 100 micrograms/ml streptomycin. $10^6$,/ml of the cells and 0.1 microgram/ml DNP-OA are cultured in microtiter plates. The suppressive effect on the IgE-producing cels is assayed in the presence of IgE-PF (i.e., the assay measures the ability of the suspected IgE-SF to counteract the effects of a known IgE-PF). A 24-hour-culture filtrate of MLN cells obtained from rats on the 14th day of Nb infection can be employed as a source of IgE-PF. Both the sample to be tested for suppressive effect and the culture filtrate containing IgE-PF are added to the DNP-OA primed cells. After 5 days of culturing with DNP-OA, the numbers of IgE-containing cells and $IgG_2$ containing cells developed in the culture are compared with those developed in control cultures that contained only DNP-OA and IgE-PF. Ig expression is determined by immunofluorescence, e.g. with labeled rabbit or goat anti-rat-IgE or -$IgG_2$ antibodies.

The peptide of Formula II at concentrations of 1 microgram/ml or 0.1 microgram/ml was found to be capable of switching lectin affinity of IgE-binding factor produced by mouse lymphocytes.

For preparing pharmaceutical compositions containing the peptides described by this invention, such peptides are combined in a mixture with preferably inert, pharmaceutically acceptable carriers. Suitable carriers and processes for their preparation are well known in the art (see, e.g., Remington's Pharmaceutical Sciences and U.S. Pharmacopeia: National Formulary, Mack Publishing Company, Easton, PA [1980]). The preferred course of administration is parenteral and can include mechanical delivery systems.

Preferably, the pharmaceutical composition is in unit dosage form. In such form, the preparation is subdivided into unit doses containing appropriate quantities of the active component. The quantity of active compound in a unit dose of preparation may be varied or adjusted from 1 μg to 100 mg, according to the particular application and the potency of the active ingredient. The composition can, if desired, also contain other therapeutic agents.

The dosages may be varied depending upon the requirements of the patient, the severity of the condition being treated, the route of administration, and the particular compound being employed. Determination of the proper dosage for a particular situation is within the skill of the art. The term "effective amount" as used herein in reference to the peptides of the invention means the amount necessary to bring about the desired therapeutic effect. It is understood that this amount varies with the circumstances of application, and that some routine experimentation may be required to determine its precise value in particular applications. Generally, treatment is initiated with smaller dosages which are less than the optimum dose of the compound. Thereafter, the dosage is increased by small amounts until the optimum effect under the circumstances is reached. For convenience, the total daily dosage may be administered in a single dose or in divided doses.

The description of the foregoing embodiments of the invention has been presented for the purpose of illustration and description. It is not intended to be exhaustive or to limit the invention to the precise forms disclosed, and obviously many modifications and variations are possible in light of the above teaching. The embodiments were chosen and described in order to best explain the principles of the invention and its practical application so as to enable others skilled in the art to best utilize the invention in various embodiments and with various modifications as are suited to the particular use contemplated.

**Claims**

I. An immunosuppressive peptide having, in the reduced form, the formula:

H - Cys - X(Val) - Cys - X(Val) - Cys - X(Leu) - X(Leu) - X(Pro) - X(Arg) - X(Tyr) - X(Pro) - Ser - X(Ala) - -
Gly - X(Val) - X(Phe) - Thr - X(Tyr) - X(Leu) - X(Asn) - Ihr - X(Lys) - X(Ile) - X(Ile) - Thr - X(Phe) - X(Asp) --
Ser - X(Val) - X(Leu) - Ser - X(Lys) - Cys - X(Ala) - OH

Formula I
   wherein:

      X(Arg) represents the group consisting of
       Arg, His, and Lys;

      X(Leu) represents the group consisting of
       Leu, Ile, Phe, and Met;

      X(Pro) represents the group consisting of
       Pro and Ala;

      X(Ala) represents the group consisting of
       Ala and Pro;

      X(Val) represents the group consisting of
       Val, Met, and Ile;

      X(Ile) represents the group consisting of
       Ile, Met, Phe, Val, Leu;
      X(Phe) represents the group consisting of
       Phe, Met, Tyr, Ile, and Leu;

      X(Tyr) represents the group consisting of
       Tyr and Phe;

      X(Asn) represents the group consisting of
       Asn and Asp;

      X(Lys) represents the group consisting of
       Lys and Arg; and

      X(Asp) represents the group consisting of
       Asp and Asn;

together with the 2-fold inserted and/or 2-fold deleted peptides of formula I;
and the partially and fully oxidised forms thereof;
      in particular a peptide wherein:

      X(Leu) represents the group consisting of
       Leu, Ile, and Met;

      X(Ile) represents the group consisting of
       Ile, Met, and Leu;

       X(Arg) is Arg;
       X(Pro) is Pro;
       X(Ala) is Ala;
       X(Val) is Val;

X(Phe) is Phe;
X(Tyr) is Tyr;
X(Asn) is Asn;
X(Lys) is Lys; and
X(Asp) is Asp.

more preferably a peptide wherein X(Leu) is Leu and X(Ile) is Ile.

2. A peptide as claimed in claim I selected from all 2-fold substituted, preferably I-fold substituted, peptides of the formula:

H - Cys - X(Val) - Cys - X(Val) - Cys - X(Leu) - X(Leu) - X(Pro) - X(Arg) - X(Tyr) - X(Pro) - Ser - X(Ala) - - Gly - X(Val) - X(Phe) - Thr - X(Tyr) - X(Leu) - X(Asn) - Thr - X(Lys) - X(Ile) - X(Ile) - Thr - X(Phe) - X(Asp) -- Ser - X(Val) - X(Leu) - Ser - X(Lys) - Cys - X(Ala) - OH.

3. A peptide as claimed in claim I selected from all 2-fold, preferably I-fold, inserted peptides derived from a peptide of the formula:

H - Cys - X(Val) - Cys - X(Val) - Cys - X(Leu) - X(Leu) - X(Pro) - X(Arg) - X(Tyr) - X(Pro) - Ser - X(Ala) - - Gly - X(Val) - X(Phe) - Thr - X(Tyr) - X(Leu) - X(Asn) - Thr - X(Lys) - X(Ile) - X(Ile) - Thr - X(Phe) - X(Asp) -- Ser - X(Val) - X(Leu) - Ser - X(Lys) - Cys - X(Ala) - OH

wherein:

X(Arg) represents the group consisting of
Arg, His, and Lys;

X(Leu) represents the group consisting of
Leu, Ile, Phe, and Met;

X(Pro) represents the group consisting of
Pro and Ala;

X(Ala) represents the group consisting of
Ala and Pro;

X(Val) represents the group consisting of
Val, Met, and Ile;

X(Ile) represents the group consisting of
Ile, Met, Phe, Val, Leu;

X(Phe) represents the group consisting of
Phe, Met, Tyr, Ile, and Leu;

X(Tyr) represents the group consisting of
Tyr and Phe;

X(Asn) represents the group consisting of
Asn and Asp;

X(Lys) represents the group consisting of
Lys and Arg; and

X(Asp) represents the group consisting of
Asp and Asn;

and each inserted amino acid residue is identical to one of the amino acid residues flanking the insertion;

in particular a peptide wherein:

X(Leu) represents the group consisting of
Leu, Ile, and Met;

X(Ile) represents the grop consisting of
Ile, Met, and Leu;

X(Arg) is Arg;

X(Pro) is Pro;
X(Ala) is Ala;
X(Val) is Val;
X(Phe) is Phe;
X(Tyr) is Tyr;
X(Asn) is Asn;
X(Lys) is Lys; and
X(Asp) is Asp.

preferably a peptide selected from all 2-fold inserted peptides derived from a peptide of the formula:

H - Cys - Val - Cys - Val - Cys - Leu - Leu - Pro - Arg - Tyr - Pro - Ser - Ala - Gly - Val - Phe - Thr - Tyr - Leu - Asn - Thr - Lys - Ile - Ile - Thr - Phe - Asp - Ser - Val - Leu - Ser - Lys - Cys - Ala - OH.

4. A peptide as claimed in claim 3 selected from all I-fold inserted peptides derived from a peptide of the formula:

H - Cys - X(Val) - Cys - X(Val) - Cys - X(Leu) - X(Leu) - X(Pro) - X(Arg) - X(Tyr) - X(Pro) - Ser - X(Ala) - - Gly - X(Val) - X(Phe) - Thr - X(Tyr) - X(Leu) - X(Asn) - Thr - X(Lys) - X(Ile)
X(Ile) - Thr - X(Phe) - X(Asp) - Ser - X(Val) - X(Leu) - Ser - X(Lys) - Cys - X(Ala) - OH;

wherein:

X(Leu) represents the group consisting of
Leu, Ile, and Met;

X(Ile) represents the group consisting of
Ile, Met, and Leu;

X(Arg) is Arg;
X(Pro) is Pro;
X(Ala) is Ala;
X(Val) is Val;
X(Phe) is Phe;
X(Tyr) is Tyr;
X(Asn) is Asn;
X(Lys) is Lys; and
X(Asp) is Asp;

in particular from a peptide of the formula:

H - Cys - Val - Cys - Val - Cys - Leu - Leu - Pro - Arg - Tyr - Pro - Ser - Ala - Gly - Val - Phe - Thr - Tyr - Leu - Asn - Thr - Lys - Ile - Ile - Thr - Phe - Asp - Ser - Val - Leu - Ser - Lys - Cys - Ala - OH.

5. A peptide as claimed in claim I selected from all 2-fold deleted peptides derived from a peptide of the formula:

H - Cys - X(Val) - Cys - X(Val) - Cys - X(Leu) - X(Leu) - X(Pro) - X(Arg) - X(Tyr) - X(Pro) - Ser - X(Ala) - - Gly - X(Val) - X(Phe) - Thr - X(Tyr) - X(Leu) - X(Asn) - Thr - X(Lys) - X(Ile) - X(Ile) - Thr - X(Phe) - X(Asp) -- Ser - X(Val) - X(Leu) - Ser - X(Lys) - Cys - X(Ala) - OH

wherein:

X(Arg) represents the group consisting of
Arg, His, and Lys;

X(Leu) represents the group consisting of
Leu, Ile, Phe, and Met;

X(Pro) represents the group consisting of
Pro and Ala;

X(Ala) represents the group consisting of
Ala and Pro;

X(Val) represents the group consisting of
Val, Met, and Ile;

X(Ile) represents the group consisting of
Ile, Met, Phe, Val, Leu;

X(Phe) represents the group consisting of
Phe, Met, Tyr, Ile, and Leu;

X(Tyr) represents the group consisting of
Tyr and Phe;

X(Asn) represents the group consisting of
Asn and Asp;

X(Lys) represents the group consisting of
Lys and Arg; and

X(Asp) represents the group consisting of
Asp and Asn;

in particular a peptide wherein:

X(Leu) represents the group consisting of
Leu, Ile, and Met;

X(Ile) represents the group consisting of
Ile, Met, and Leu;

X(Arg) is Arg;
X(Pro) is Pro;
X(Ala) is Ala;
X(Val) is Val;
X(Phe) is Phe;
X(Tyr) is Tyr;
X(Asn) is Asn;
X(Lys) is Lys; and
X(Asp) is Asp;

more preferably a peptide selected from all 2-fold deleted, preferably I-fold deleted, peptides derived from a peptide of the formula:

H - Cys - Val - Cys - Val - Cys - Leu - Leu - Pro - Arg - Tyr - Pro - Ser - Ala - Gly - Val - Phe - Thr - Tyr - Leu - Asn - Thr - Lys - Ile - Ile - Thr - Phe - Asp - Ser - Val - Leu - Ser - Lys - Cys - Ala - OH.

6. A peptide as claimed in claim 5 selected from all I-fold deleted peptides derived from a peptide of the formula:

H - Cys - X(Val) - Cys - X(Val) - Cys - X(Leu) - X(Leu) - X(Pr0) - X(Arg) - X(Tyr) - X(Pro) - Ser - X(Ala) - - Gly - X(Val) - X(Phe) - Thr - X(Tyr) - X(Leu) - X(Asn) - Thr - X(Lys) - X(Ile) - X(Ile) - Thr - X(Phe) - X(Asp) -- Ser - X(Val) - X(Leu) - Ser - X(Lys) - Cys - X(Ala) - OH;

wherein:

X(Leu) represents the group consisting of
Leu, Ile, and Met;

X(Ile) represents the grop consisting of
Ile, Met, and Leu;

X(Arg) is Arg;
X(Pro) is Pro;
X(Ala) is Ala;
X(Val) is Val;
X(Phe) is Phe;
X(Tyr) is Tyr;
X(Asn) is Asn;
X(Lys) is Lys; and

11

X(Asp) is Asp.

7. A peptide as claimed in claim 5 or claim 6 selected from all I-fold inserted peptides derived from a peptide of the formula:

H - Cys - Val - Cys - Val - Cys - Leu - Leu - Pro - Arg - Tyr - Pro - Ser - Ala - Gly - Val - Phe - Thr - Tyr - Leu - Asn - Thr - Lys - Ile - Ile - Thr - Phe - Asp - Ser - Val - Leu - Ser - Lys - Cys - Ala - OH;

wherein the inserted amino acid residue is identical to one of the amino acid residues flanking the insertion.

8. A pharmaceutical composition for suppressing the IgE immune response comprising a therapeutically compatible carrier and an effective amount of a peptide of claims I, 3, 5 or 7.

9. The pharmaceutical composition of claim I8 wherein said peptide is selected from the peptides defined by claims 2, 4 or 6.

I0. A process for the preparation of a peptide of formula I defined in claim I, or of a 2-fold inserted and/or 2-fold deleted peptide thereof, or of a partially or fully oxidised form thereof, which comprises the steps of:

I) providing a base-support with a functional group to which the first amino acid is anchored;

2) coupling the next amino acid, protected, to build up the polypeptide chain,

3) deprotecting as necessary said next amino acid;

4) repeating steps 2) and 3) until the full polypeptide chain is assembled; and

5) cleaving the complete polypeptide chain from the base support.